# EUROPEAN PATENT SPECIFICATION

(11) **EP 2 948 132 B1**
(45) Date of publication and mention of the grant of the patent: **19.08.2020**
(21) Application number: 14743490.6
(22) Date of filing: 23.01.2014
(51) Int. Cl.: A61K 9/127, A61K 49/18, A61K 49/00, A61K 51/08, A61K 51/12

(54) **METHOD FOR DIAGNOSING OR TREATING TUMORS USING SPHINGOMYELIN CONTAINING LIPOSOMES**
VERFAHREN ZUR DIAGNOSE ODER BEHANDLUNG VON TUMOREN UNTER VERWENDUNG SPHINGOMYELINHALTIGER LIPOSOME
PROCÉDÉ POUR DIAGNOSTIQUER OU TRAITER DES TUMEURS AU MOYEN DE LIPOSOMES CONTENANT DE LA SPHINGOMYÉLINE

(30) Priority: 24.01.2013 US 201361756116 P
(43) Date of publication of application: 02.12.2015
(73) Proprietor: Memorial Sloan Kettering Cancer Center, New York, NY 10065 (US)
(72) Inventor: PENATE-MEDINA, Tuula, New York, New York 10065 (US); PENATE-MEDINA, Oula, New York, New York 10065 (US); LARSON, Steven M., New York, New York 10065 (US); GRIMM, Jan, New York, New York 10065 (US); THOREK, Daniel L. J., New York, New York 10065 (US); KOLESNICK, Richard N., New York, New York 10065 (US)
(74) Representative: Kordel, Mattias
(86) International application number: PCT/US2014/012802
(87) International publication number: WO 2014/116866

(56) References cited:
- WO-A1-00/59473
- WO-A1-2005/002546
- WO-A2-03/000227
- BR-A2- PI1 003 415
- US-A1- 2003 147 945
- US-A1- 2011 135 571
- US-A1- 2011 142 763
- US-A1- 2011 165 084
- US-B2- 8 110 217
- WEBB M S ET AL: "Sphingomyelin-cholesterol liposomes significantly enhance the pharmacokinetic and therapeutic properties of vincristine in murine and human tumour models", BRITISH JOURNAL OF CANCER, NATURE PUBLISHING GROUP, GB, vol. 72, no. 4, 1 January 1995 (1995-01-01), pages 896-904, XP002667117, ISSN: 0007-0920
- ODA M ET AL: "Hemolysis induced by Bacillus cereus sphingomyelinase", BIOCHIMICA ET BIOPHYSICA ACTA (BBA) - BIOMEMBRANES, ELSEVIER, AMSTERDAM, NL, vol. 1798, no. 6, 1 June 2010 (2010-06-01) , pages 1073-1080, XP027035824, ISSN: 0005-2736 [retrieved on 2010-03-07]
- HIROFUMI SAWAI ET AL: "Ceramide and sphingomyelinases in the regulation of stress responses", CHEMISTRY AND PHYSICS OF LIPIDS., vol. 102, no. 1-2, 1 November 1999 (1999-11-01), pages 141-147, XP055295996, IR ISSN: 0009-3084, DOI: 10.1016/S0009-3084(99)00082-1
- MARCO TLIO R GOMES ET AL: "Determination of sphingomyelinase-D activity ofvenoms in sphingomyelin/cholesterol liposomes containing horseradish peroxidase", TOXICON, ELMSFORD, NY, US, vol. 57, no. 4, 2 January 2011 (2011-01-02), pages 574-579, XP028175513, ISSN: 0041-0101, DOI: 10.1016/J.TOXICON.2011.01.001 [retrieved on 2011-01-12]
- Shihong Li ET AL: "Novel Multifunctional Theranostic Liposome Drug Delivery System: Construction, Characterization, and Multimodality MR, Near-Infrared Fluorescent, and Nuclear Imaging", Bioconjugate Chemistry, vol. 23, no. 6, 20 June 2012 (2012-06-20), pages 1322-1332, XP055297906, ISSN: 1043-1802, DOI: 10.1021/bc300175d
- Madaswamy S Muthu ET AL: "Theranostic liposomes for cancer diagnosis and treatment: current development and pre-clinical success", Expert Opinion on Drug Delivery, vol. 10, no. 2, 15 October 2012 (2012-10-15), pages 151-155, XP55424815, GB ISSN: 1742-5247, DOI: 10.1517/17425247.2013.729576

## Description

### FIELD OF THE INVENTION

This invention relates to sphingomyelin-containing liposomal systems and methods using the liposomal systems for delivering a therapeutic agent and an imaging agent to a cell sample under *in vitro* conditions.

### BACKGROUND OF THE INVENTION

Ceramide formation can be induced by exposure to chemotherapy via *de novo* and sphingomyelinase (SMase) pathways and by ionizing radiation, which produces ceramide at the plasma membrane by activating acid SMase. Ionizing radiation-induced ceramide functions as a second messenger to initiate apoptosis. Through its capacity to induce programmed cell death (apoptosis), ceramide can function as a potent tumor suppressor lipid. Ceramide can also limit cancer cell proliferation by blocking cell cycle transition. Ceramide can also instigate autophagic responses in cancer cells; however, these may yield survival or lethal outcomes.

Extracellular release of acid sphingomyelinase (ASMase, acid SMase or SMase) in response to cell stress, such as toxins, chemotherapeutics, the presence of inflammation or substantial UV or X-ray irradiation, initiates a rapid ceramide-mediated endothelial cell injury response leading to endothelial apoptosis (as well as in lesser amounts in other tissues and tumor cells). Released Acid SMase hydrolyzes endothelial surface sphingomyelin to generate the pro-apoptotic second messenger ceramide. This presents opportunities for novel approaches to selectively target therapies to sites of cell stress, particularly the vascular component of tumor response.

U.S. Patent Nos. 7,288,534 and 6,541,462 (Modrak) propose use of sphingomyelin lipids for treatment of cancer and rheumatoid arthritis. U.S. Patent No. 8,110,217 (Chancellor) concerns treatment of hyperactive bladder disorder with sphingomyelin liposomes. Barenholtz (WO 2010/041255) discusses liposomes that contain sphingomyelin to increase shelf life of liposomes encapsulating active agents such as chemotherapy or analgesics.

At the moment there are several methods to detect apoptosis: DNA laddering, Annexin V, among others. However, there is a need for a reliable method for *in vivo* apoptosis detection. Only Annexin V has been used for *in vivo* apoptosis detection, as reported in some articles, but the results seem mixed. This may be because Annexin V detects a single phospholipid, phosphatidyl serine. The ability to selectively and accurately delineate the activation of the acid SMase pathway in living specimens achieves the ability to visualize and quantify a key pathway of response to cell stress.

While other researchers have explored various uses of sphingomyelin and liposomes including sphingomyelin, a need exists for a molecular imaging probe that can act with sufficient specificity to interact with ASMase to provide information about endothelial apoptosis, while also providing a liposomal releasing system for imaging agents, such as fluorescent dyes chemotherapeutics and MR imaging agents (both gadolinium chelates and heavy water, D₂0).

### SUMMARY OF THE INVENTION

The present invention provides a method to probe (image) ASMase's specific and highly restricted function in the cases when ASMase is up-regulated, available outside of cells and active. This combines the imaging function and the ASMase activation function so that only the intended location will be ASMase active and capable to perform the targeting and release function and monitor the release function, by accessing, imaging and manipulating this biology in the proximity of released ASMase. This method utilizes the SM-liposome in four steps: Target Image-Activation-Release-Follow-up image (TIARA). The innovation can be used by utilizing some of the four steps.

The invention provides a liposomal system, comprising: (a) a first layer comprising sphingomyelin or a derivative thereof having modified or shortened side chains;
(b) a second layer comprising sphingomyelin or a derivative thereof having modified or shortened side chains; wherein the first and second layers form a bilayer liposome defining a hydrophilic interior compartment and a hydrophobic bilayer compartment;
(c) at least one therapeutic agent in the hydrophilic interior compartment or the hydrophobic bilayer compartment; and
(d) at least one imaging agent in the hydrophilic interior compartment or the hydrophobic bilayer compartment; wherein the imaging agent is: (i) quenched Cy 5,5, (ii) a DNA segment comprising a DNA Beacon containing a hairpin loop with a CY7-Dabcyl quenching pair or other fluorophor quencher pair, or (iii) gadolinium and deuterium (D20).

The invention additionally provides a method for delivering a therapeutic agent and an imaging agent, comprising: contacting the liposomal system according to the present invention with a cell sample under *in vitro* conditions wherein SMase present in or released by the cell sample can hydrolyze the sphingomyelin in the liposomal system to release therapeutic agent and the imaging agent from the liposomal system.

Finally, the ability to co-mix imaging and therapeutic agents enables the ability to readout or visualize or infer the release of a drug agent. This so called theranostic strategy is another advantage of the approach herein.

### BRIEF DESCRIPTION OF THE DRAWINGS

Further objects and advantages of the invention will be apparent to those of ordinary skill in the art upon reviewing the following description of the preferred embodiments taken in connection with the following drawings in which:

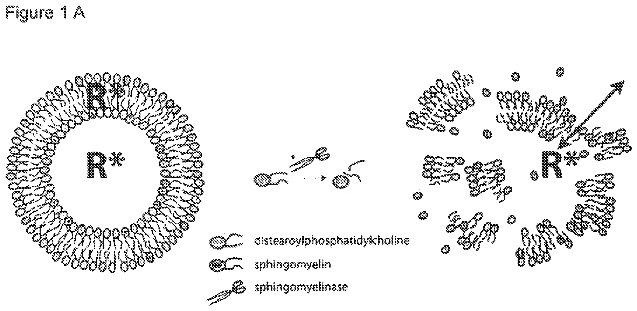

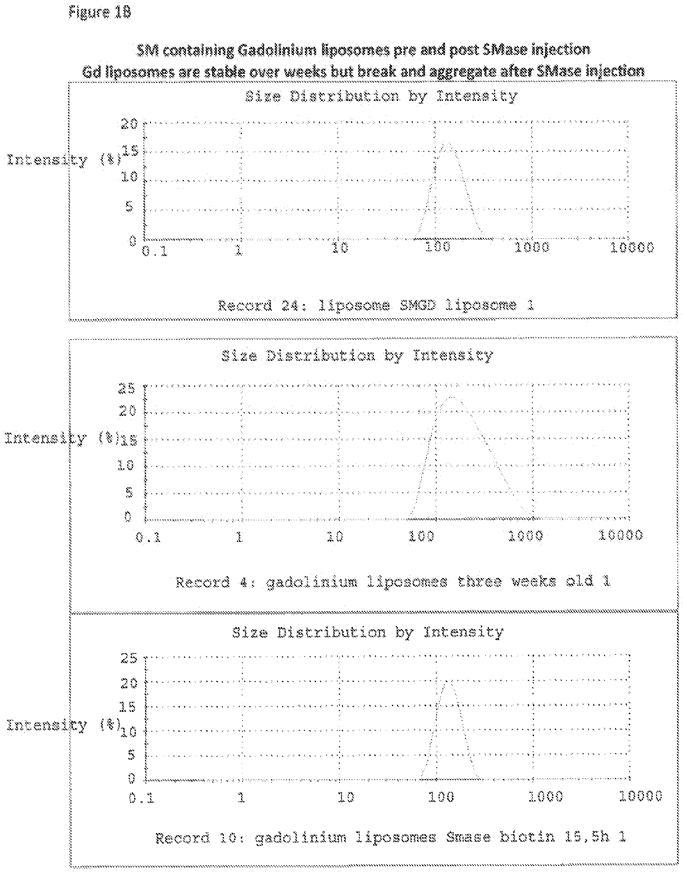

Figure 1A shows a schematic of the present invention. Liposomes containing a reagent (R*) which may be a combination of a fluorophore, a therapeutic agent, a contrast agent, or a DNA segment, while Figure 1B is a size distribution of sphingomyelin liposomes by dynamic side scattering, as synthesized by manual extrusion.
Figure 2A is a diagram of liposomal CY7-Dabcyl hairpin encapsulated in SM liposomes released by SMase (0.1 units/µl in one ml well) to outside medium containing target DNA; when target DNA hits the hairpin, the hairpin is released giving free Cy7 fluorescence;
Figure 2B shows Cy 5,5 is quenched when packed in liposomes; and ASMase treatment increases the signal by rupturing the liposome and releasing the dye. Heat inactivated ASMase is not able to rupture the liposome.

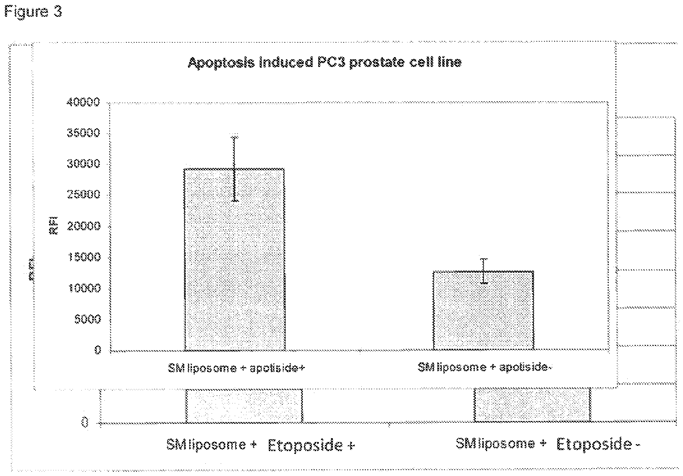

Figure 3 shows apoptosis activation based detection of SM Liposomes containing Cy 5,5.

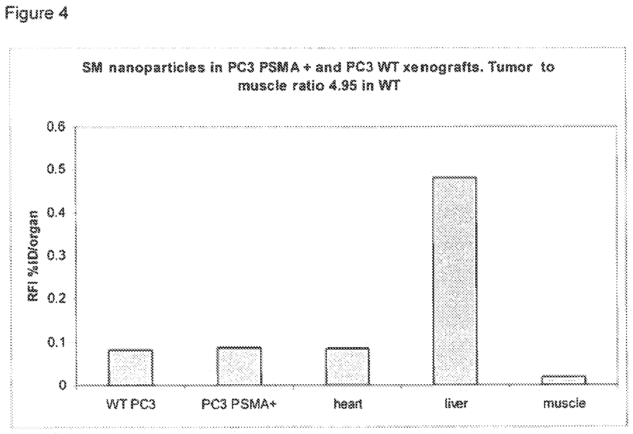

Figure 4 shows biodistribution of SM liposome carrying Cy 5,5 in PC3 xenograft mice at 2h.

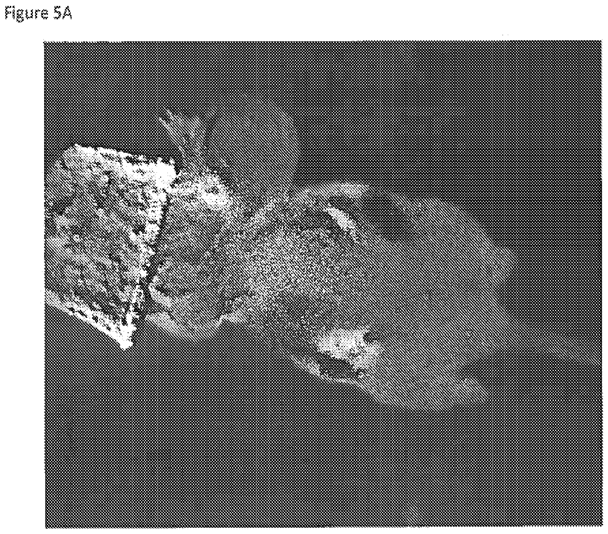

Figure 5A is fluorescence image of necrotic tumor K562 targeted with PC-liposome (without SM lipid) with encapsulated Cy 5,5 administered through tail vein iv-injection.

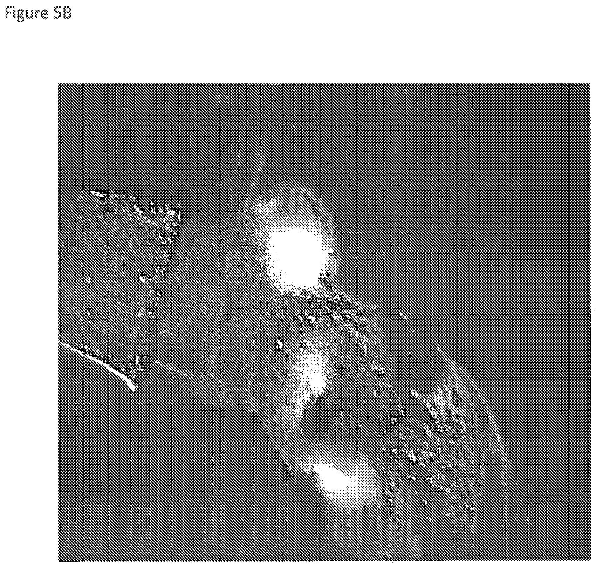

Figure 5B is a fluorescence image of necrotic tumor K562 targeted with SM liposomes with encapsulated Cy 5,5 administered through tail vein iv-injection.

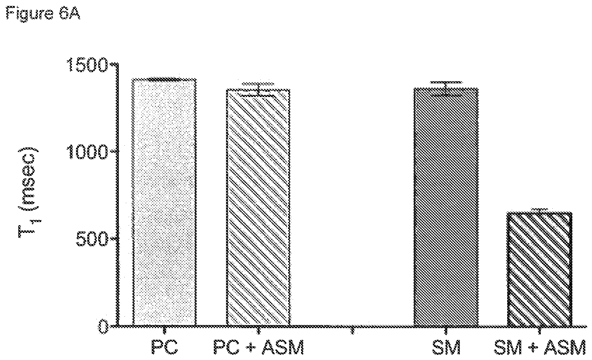

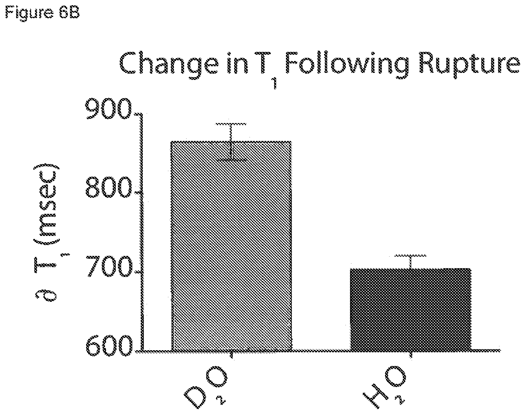

Figure 6A is a demonstration of the specific activation of Gadolinium-loaded liposomes. The PC liposomes do not contain sphingomyelin lipids, while the SM group contains the sphingolipid. Upon addition of the activating enzyme, Acid SMase, the particles are perforated, and the T1-relaxation signal decreases (brightens) only in the SM liposomes. Figure 6B shows the augmented contrast that can be achieved when the interior of the liposome contains Gadolinium and deuterium (D2O) as compared to conventional liposomes filled with H2O.

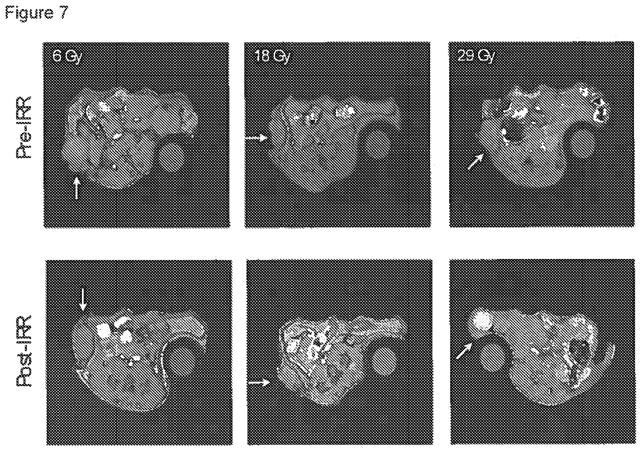

Figure 7 shows the activation of the particles in vivo to read out the response of cells to external stress, through release of Acid SMase, for diagnostic purposes. Here, in the top row we have images of animals (with tumors pointed to with arrow) with a tube-of-water standard. These mice have already been injected with the gadolinium loaded sphingomyelin containing liposomes. On the bottom row, the same mice are shown, following external beam irradiation at (left to right, 6 Gy, 18 Gy or 29 Gy). A dose dependent increase in signal, following destabilization of the liposome by Acid SMase.

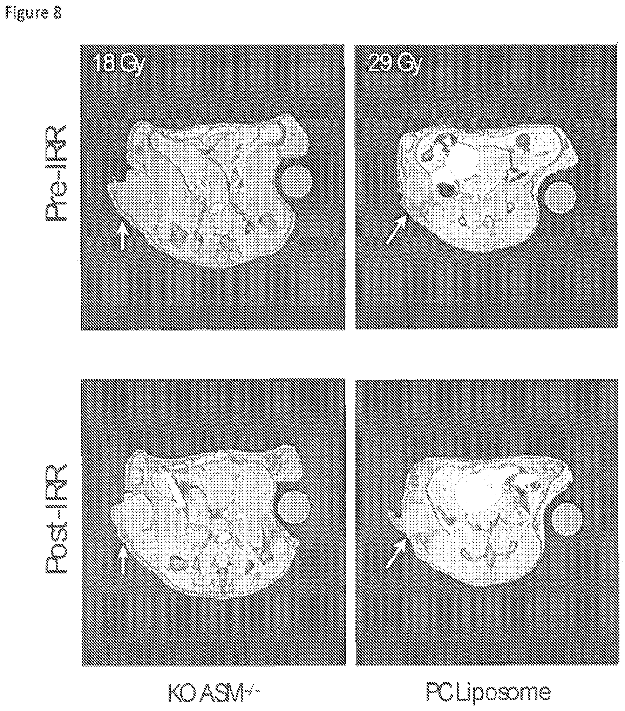

Figure 8 are representative MR images that demonstrate the specificity of the sphingomyelin containing liposomes for activation by a unique biological phenomenon: release of Acid SMase. Again, white arrows point to the tumor, with pre- and post-irradiation images on the top and bottom rows respectively. On the left are sphingomyelin containing liposomes that have been administered to Acid SMase knockout mice. These mice do not express the enzyme Acid SMase and it is shown that the presence of this enzyme is required for the activation of the MR-contrast loaded liposome. On the right are representative images of a normal (Acid SMase producing) mouse which has been injected with a non-sphingomyelin containing liposome. This liposome cannot be activated and the MR signal in the liposome does not change.

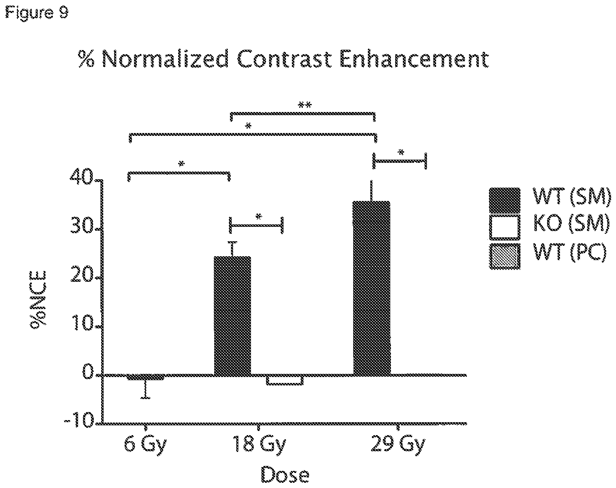

Figure 9 demonstrates the diagnostic specificity and accuracy of MR-contrast agent loaded sphingomyelin-liposomes for detection of the Acid SMase mediated cell stress response following X-ray irradiation.

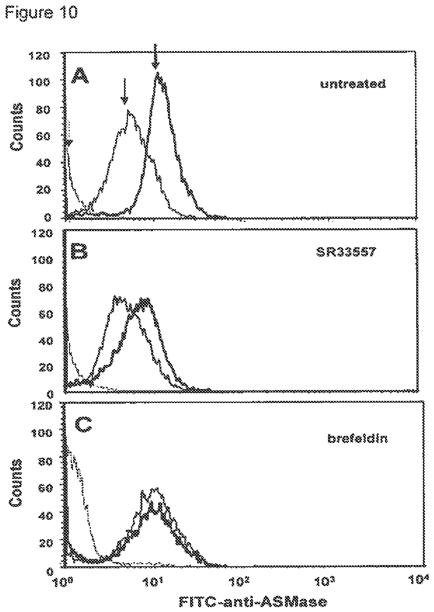

Figure 10 are graphs showing transport of SMase transport to the outer leaflet of the plasma membrane during cell apoptosis.

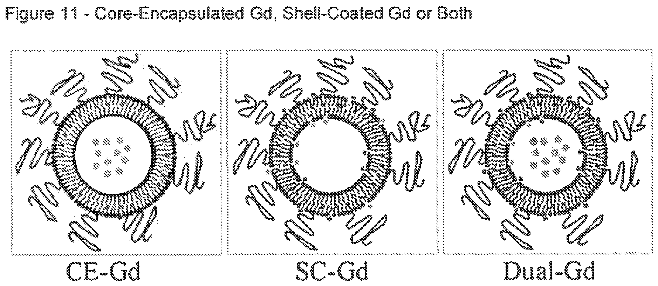

Figure 11 is a diagram of different modes of attachment of gadolinium to a liposome for use in one embodiment of the present invention.

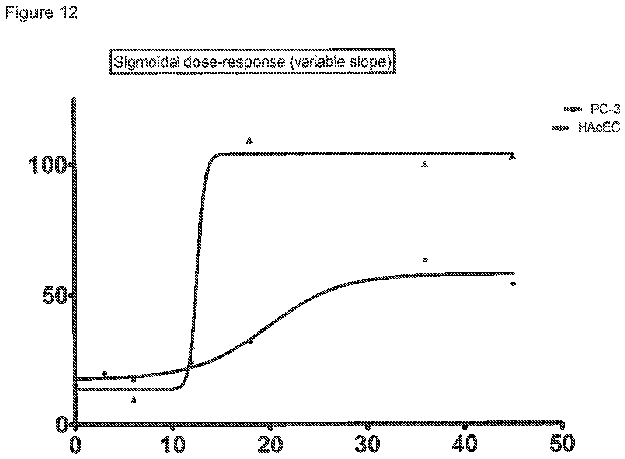

Figure 12 is a graph showing leakage of Indocyanine green (as a model of release of therapeutic compounds) from SM liposomes after 1h incubation with human aortic endothelial cells (HAoEC) or PC-3 prostate cells after having been irradiated by different doses.

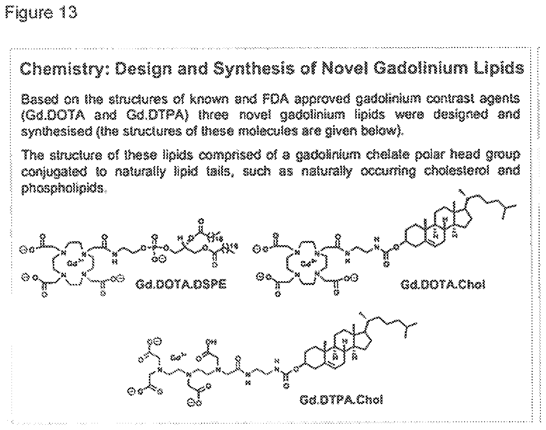

Figure 13 shows structure and synthesis of gadolinium lipids for use in one embodiment of the present invention.
Figure 14A shows preparation of liposomal nano-formulations; Figure 14B purification liposomes by size exclusion column chromatography; Figure 14C size distribution of SM liposomes by dynamic light scattering.

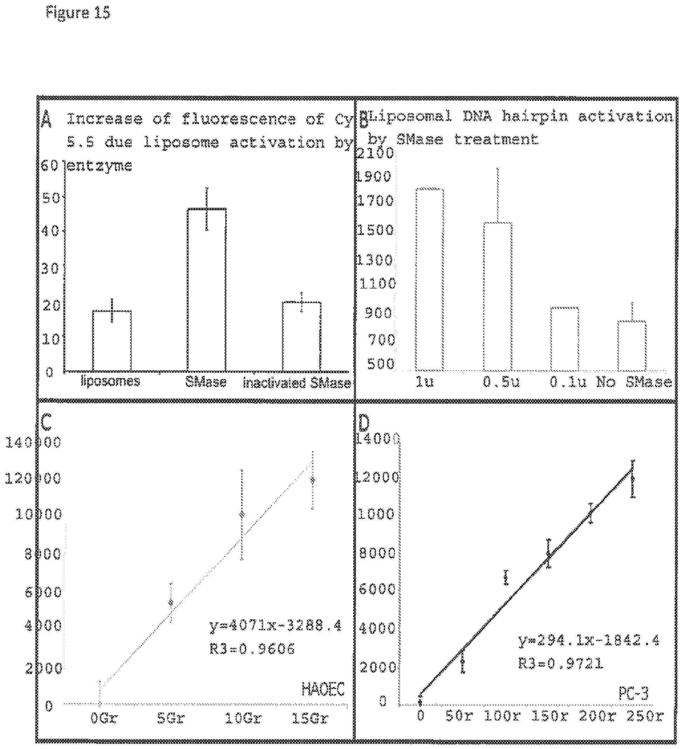

Figure 15A shows fluorescence of Cy 5.5. Fluorescence of Cy 5.5 is quenched when in packed in liposomes. SMase treatment increases the signal by rupturing the liposome and releasing the dye. When Cy5.5 is released, the fluorescence intensity is increased. Heat inactivated SMase is used as control. Figure 15B shows Liposomal CY7-Dabcyl hairpin encapsulated in SM liposomes. Released by SMase in three SMase concentrations (1 u unit, 0.5 u units and 0.1 u units/ml) to the medium outside containing the target DNA. When the target hits the hairpin, the hairpin opens and the Cy7 is unquenched. Figures 15C/D show dose responsive cell stress detection. SMase secretion after HAoEC (15C) and PC-3 cells (15D), respectively are irradiated with various doses. SMase is detected by using SMase fluorescence kit.

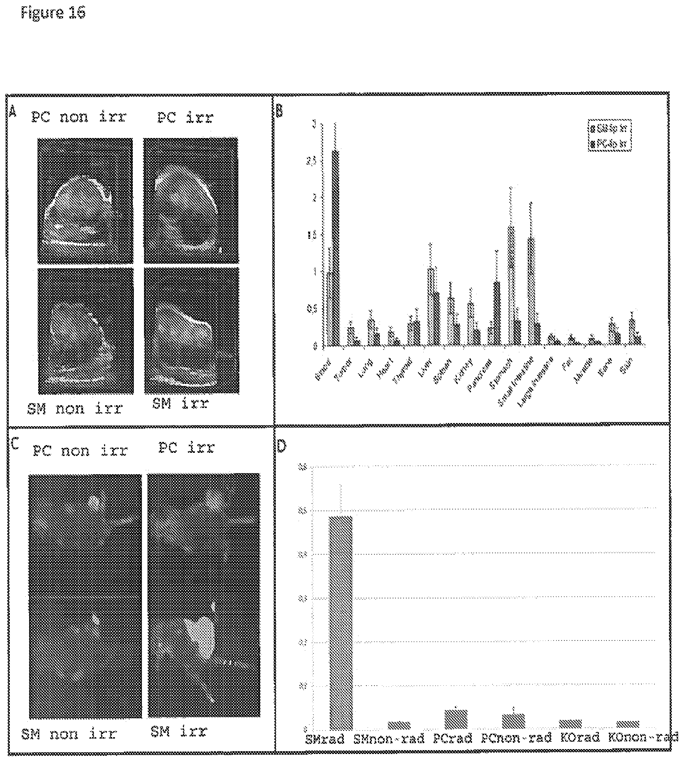

Figure 16A shows ultrasound morphology analysis of the PC-3 tumors, with sizing of tumors using ultrasound. Figure 16B shows 2h Biodistribution of SM liposome vs. PC liposome in mice with irradiated tumors. Radiolabeled BSA was used as the payload inside of the liposomes. Figure 16C shows comparison of SM-liposomes with PC-liposomes containing Alexa Fluor 680 fluorophores in irradiated and non-irradiated tumors. The irradiated tumors are marked by an arrow. Images were taken immediately after irradiation, i.e. 30 minutes after injection and imaging was performed using a Maestro optical camera using spectral unmixing. Figure 16D shows PC-3 tumor xenograft mice, area of interest (ROI) at irradiated tumor site and control ROI at the same site at the other hind leg with similar non irradiated tumor site, analyses (max relative fluorescence intensity RFI of the site was used.
Figure 17 (two images on the top) shows optical imaging of irradiated nude mice bearing PC-3 tumors one animal on the bottom shows optical image of SMase knockout mouse scid mouse bearing PC 3 tumor in both of the back flanks. Mice were injected with Alexa Fluor 680 fluorophores-containing liposomes following irradiation with 20 Grays. The irradiated tumors are marked by an arrow; the tumor on the opposite leg is used as a control. Images were taken immediately after irradiation, i.e. 30 minutes after injection; imaging was performed using a Maestro optical camera and using spectral unmixing.

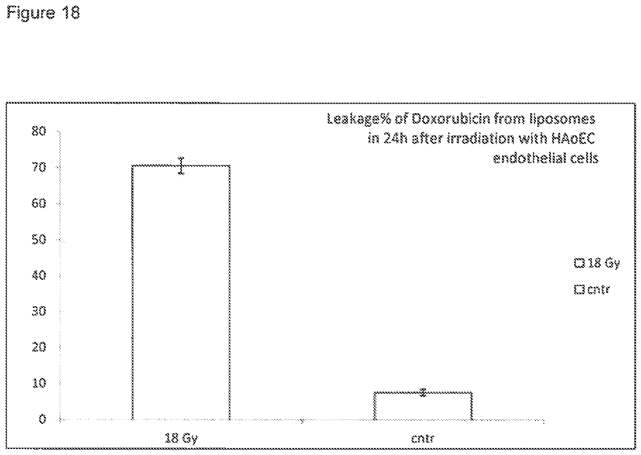

Figure 18 shows percentage of leakage in 24 hours of Doxorubicin from liposomes after irradiation of HAoEC endothelial cells (versus control).
Figure 19 shows percentage of leakage Doxorubicin from liposomes after one hour induction with irradiated PC-3 cells.
Figure 20 is a graph of percent leakage of liposomes vs. radiation dosage in HAoEC and PC-30 cells.
Figure 21 is a graph of percent leakage of liposomes versus incubation time with irradiated HAoEC and PC-3 cells.

### DETAILED DESCRIPTION OF THE PREFERRED EMBODIMENTS

The present invention provides a method that can amplify the detection of a biological response mechanism to stress by using enzymatic activation. The enzyme will cleave thousands of sphingomyelins into ceramides in the surface of the carrier liposome, and as a result cause the rupture or perforation of the liposomes. This change in the integrity of the liposomal container leads to the release of the payload such as an imaging or therapeutic agent to the extracellular or intracellular compartment. This enzymatic activation of the nanoparticle is induced following cell stress (e.g. high dose X-ray irradiation, ultraviolet light exposure, high energy ultrasound; Czarnota et al, Tumor radiation response enhancement by acoustical stimulation of the vasculature, PNAS, 2012) which triggers release of the sphingolipid hydrolase Acid SMase.

Detection of this biological event is amplified by coupling the payload of liposomal nanoparticles to fluorophores or MRI contrasting agents or DNA that will intensify when outside the liposome. The method may be used *in vitro* in test tube assays, and *in vitro* in cell culture assays to assay cell stress response including those leading to apoptosis.

In one embodiment of the invention, naturally occurring sphingomyelin can be used in forming the liposomes of the present invention, such as is available from Sigma Chemical, St. Louis, Mo. The sphingomyelin can be synthesized according to known methods, see U.S. Patent No. 6,541,462. According to the invention, the sphingomyelin can be a derivative or metabolite thereof, such as with modified or shortened side chains.

The liposome forming lipid for use in the present invention may include, without limitation, various types of phospholipids, glycolipids, and glycerophospholipids. The lipids may be cationic, monocationic, or polycationic, provided they are compatible with sphingomyelin and the active agent or indicator species used in practicing the invention.

A variety of classes of chemotherapeutics (approved and currently in trial) that have been loaded into the nanoparticles for release following cell stress include without limitation: Cis-diammineplatinum(II), doxorubicin hydrochloride, marimstat, tissue necrosis factor alpha, paclitaxel, curcumin, prinomastat, ST638, SKI246, dasatnib and pro-apoptotic peptides. The releasing ASMase was stimulated by cells following irradiation. The amount of drug released was measured by absorption and fluorescent imaging signal (from co-loaded dye).

Previously it has been shown that sphingomyelinase ruptures giant liposomes if it comes in contact therewith. It has recently been demonstrated by using LUVs that in contrast to sphingomyelin, ceramide forms microdomains in both fluid and gel state phosphatidylcholine membranes. Subjecting sphingomyelin containing GUVs or liposomes to the action of externally applied sphingomyelinase resulted first in the rapid formation of ceramide-enriched microdomains, with subsequent formation of "endocytotic" vesicles in the internal cavity of the giant liposome (Holopainen et al, J. Biol. Chem. (2000), v. 275, No. 22, pages 16484-89; Nurminen et al. J. Am. Chem. Soc. (2002), 124, 12129-34). It has been previously shown that sphingomyelinase can induce vectorial budding of small vesicles from either the inner or outer leaflet of a single-walled giant vesicle (diameter >100 µm), depending on to which side of the bilayer the enzyme is added (Holopainen *et al*.). It has been demonstrated that "endocytosis" can be induced in GUVs also by LDL. Evidence for non-receptor-mediated uptake by rat luteal cells of LDL cholesterol without concomitant apoB-100 ingestion has been documented (Holopainen et al.). Recently, aggregated LDL has been shown to enter the cells by surface-connected compartments by a mechanism not involving the LDL receptor.

A variety of cell types present in atherosclerotic lesions secrete sphingomyelinase, thus suggesting a possible role for this enzyme in the development of atherosclerosis so this imaging and targeting device could be possibly used also for diagnostics imaging and treatment of these diseases.

It has been also shown that apoptotic cells transport sphingomyelinase in the outer leaflet of the apoptotic cell, a condition that will not in normal cells. It is important and desirable to know the rate of apoptosis after a treatment for example, of radiation. Apoptosis after radiation of the tumor cells has been shown by Rotolo et al. (Rotolo, J.A., et al., J. Biol. Chem. (2005) Vol. 280, No. 28, pages 26425-26434; Charruyer, A. et al., J. Biol. Chem. (2005); 280: pages 19196-19204).

Targeted drug delivery is possibly the most important goal in pharmaceutics because strictly localizing a drug's pharmacological activity to the site or organ of action, decreases the required drug dose and systemic toxicity, and increases the treatment efficacy. However, targeting is especially challenging when targets are difficult to reach because of anatomic reasons, particularly by poor blood supply, or when targets have a high drug resistance. In addition, despite recent advances in nanoparticle probe development for biomedicine, the translation of targeted diagnostic particle platforms remains challenging. Nanostructures most commonly used for drug delivery in the clinic include liposomes and micelles. Nanoparticle-based materials currently under evaluation in oncology clinical trials are largely non-targeted drug delivery vehicles. EPR effect is usually utilized in order to reach tumor sites but there is no good controlled release system that can unload the drug cargo at the clinical use at the moment. There are thermal activation but it has problems because tight thermal range between normal body temperature, phase transition temperature and tissue harming temperature. As drug delivery systems are complicated it makes sense to test the drug delivery system by using imaging techniques in order to evaluate the efficacy of the system and for using this data for development of the delivery systems and for tumor model selection and later stages for patient selection as well and have true theranostic approach. In addition to satisfy critical safety benchmarks, tumor-selective diagnostic probes need to elucidate targeted interactions with the microenvironment and their effects on biological systems. Several key factors that limit the translation of tumor-selective diagnostic probes have been identified: sub-optimum pharmacokinetics; *in vivo* probe nanotoxicity (4); and evolving regulatory considerations. To address such issues and comply with regulatory and clinical practice guidelines, rigorous quantitative imaging approaches and analysis tools, such as PET, will be essential for evaluating a variety of probes undergoing preclinical testing or transitioning into early phase clinical trials. By attaching a radiolabel to the particle surface, the clinical potential of these molecularly targeted probes can be sensitively and comprehensively defined according to the following set of diagnostic criteria: favorable distribution and targeting kinetics; extended circulation (blood residence) times; pronounced tissue signal amplification; and improved tumoral penetration.

Molecular imaging is critical to the development and success of targeted molecular medicine because it allows for early detection of diseases; monitoring of drug efficacy; and identification of disease. Therefore, there is a strong need for the development of nano probes for drug targeting, functional imaging, and targeted therapy.

Apoptosis detection is crucial for monitoring therapy outcomes in cancer. There are currently several methods of detecting apoptosis including DNA laddering, Annexin V, and caspase-activated compounds. However, there is no good method for detecting apoptosis *in vivo.* Annexin V alone has been used in the *in vivo* apoptosis detection in some articles but the results have been mixed. This is not surprisingly because Annexin V is only detecting phospholipid phosphatidyl serine. Thus, means that could accurately detect and amplify the detection of the target molecule by using enzymatic activation when in contact with the apoptotic cells would be greatly needed.

Sphingomyelin (SM) and ceramide are among the lipids that have a special role in cells during cell permeabilization, intravasation, apoptosis and domain formation. Ceramide is normally not located on the outer surface. One of the enzymes that control the SM ceramide equilibrium is Sphingomyelinase (SMase). SMase hydrolyzes the head group of SM resulting phosphocholine and ceramide. This ceramide can form domains that are then internalized inside a cell.

In addition, SMase ruptures liposomes if in contact. It has recently been demonstrated by using giant unilamellar vesicles (GUV) that when SMase is in contact whit liposomal SM, ceramide forms which leads microdomains in both fluid and gel state phosphatidylcholine membranes. Immobilized SMase in a latex particle applied externally to SM containing GUVs resulted in the rapid formation of ceramide-enriched microdomains, with subsequent formation of "endocytotic" vesicles in the internal cavity of the giant liposome (Holopainen et al, Nurminen et al).

It has also been shown that apoptotic cells transport SMase in the outer leaflet of the apoptotic cells, a condition that will never occur in healthy cells. Because apoptosis is a process that occurs in the tumors spontaneously and is increased after chemotherapy or radiotherapy, it would be helpful to know the rate of apoptosis after a treatment to have a measure for therapy efficacy. Apoptosis after radiation of the tumor cells has been shown by Rotolo et al.

Here we describe a method for accurate SMase detection by using liposomes that can be activated by SMase. In the case of apoptosis or membrane rupture, SMase is transported from the inner leaflets of the cells to their outer leaflets. We suggest that SMase transportation will also occur in the acute stress caused by irradiation of the cells and that this phenomena can be used to site specific disruption and targeting of the SM-containing liposomes. We also show that the PC-3 cell line and HAoEK cell line secretes SMase in a dose dependent manner after irradiation.

Because the versatility of our approach, the tumor homing ability of tumor-selective diagnostic probes will be tested using optical, MRI, SPECT/PET, and performing biodistribution studies. Targeted nanoparticles will be compared to non-targeted nanoparticles; targeting studies will be performed on the cell lines and tumor models that do not express the receptors for binding; and pharmaco-dynamic and pharmaco-kinetic parameters will be evaluated. Based on these results a cancer drug containing nanoparticles can be created.

We suggest that this site targeted liposomal delivery can be used for drug delivery in all possible conditions where cell stress induces SMase secretion. In drug delivery this will create a delivery system that will bring the drug available only to the stressed cells in the acute need of the drug. An advantage of the proposed system is the possible amplification of the signal since one enzyme will cleave thousands of SMs into ceramides in the surface of the carrier liposome. As a result the liposomes rupture, and release their payload to the extracellular or intracellular compartment. This enzymatic triggering is further amplified by coupling the payload of liposomal nanoparticle to fluorophores or MRI contrast agent or DNA that will intensify when they are outside the liposome. Here we show in vitro test tube, in vitro cell culture assays and in vivo some possible applications of this versatile method.

The following experimental section is meant to illustrate but not limit the practice and scope of the invention.

### EXPERIMENTAL SECTION

### EXAMPLE I

### Liposome formulations

### Sphingomyelinase from B. cereus from Sigma

Liposomes were made from a lipid mixture (Avante Polarlipids) from a total of 20 uM of lipids in 4/3/3 DSPC/Cholesterol/Sphingomyelin in mol/mol ratios. Liposomes were prepared according to the technique of Medina OP *et al.* Lipids stored in chloroform were pipetted to a round bottomed flask, dried under nitrogen and lyophilized for 2 h to remove trace amounts of chloroform. Lipids were allowed to hydrate for 30 min in 60 C buffered water solutions containing either Cy 5,5 or Magnavist gadolinium solution, or hairpin DNA. Extrusion was performed 11 times through 100 nm polycarbon membrane by using small volume extruder. Liposomal tracer was purified from the unbound tracer by PD 10 column or dialysis overnight. Liposomes were controlled by liposome size measurements by using dynamic light scattering (Malvern).

### In Vitro Cell Experiments

Human Aortic Endothelial Cells (HAoEC) and PC-3 prostate tumor cells effect to SM-liposomes with the radiation and without radiation was tested by exposing (0,5 million) cells in a cell culture flask with cell growth medium to radiation. After radiation SM-liposomes containing indo cyanine green (ICG) was added to the medium and incubated for an hour. The medium was removed to a dialysis membrane (Snake skin cut of 10 kDa) and placed the tubes in PBS buffer containing 50 ml falcon tubes, incubated overnight and measured next day how much was leaked from the liposomes to the medium and passed to the other side of the membrane. Absorbance from both sides of the membrane (abs 800 nm) was measured and leakage % ((Abs other side/Abs initial side)^{∗}100) calculated. The results show that the radiation has effect on both the PC-3 cells and the HAoEC cells but more to the HAoEC.

### Fluorescence Experiment

Liposomes containing Cy 5,5 were, after purification, incubated with sphingomyelin with different unit amounts and with heat inactivated sphingomyelin. Results were collected by using a Tecan sapphire plate reader with different time points.

Cell experiments were done by using the therapeutic agent epotiside 300 µM, on an induced PC3 prostate cell line. Etoposide was introduced to starved PC3 cells. After 72 h incubation with Etoposide, the cells were at peak of apoptosis and Cy 5,5 containing sphingomyelin liposomes were introduced to the cells. After six hours of incubation the fluorescence was read by using a Tecan Sapphire plate reader. Cells without epotiside treatment were used as a control. Also, cell experiments were performed where instead of Cy 5,5, liposomal DNA beacon containing a hairpin loop with a CY7-Dabcyl quenching attached thereto pair was used. Beacon released by SMase to outside medium containing target DNA released the hairpin and Cy7, which when no longer quenched by Dabcyl, gave a signal that was measured with a Tecan Sapphire plate reader.

Mouse experiments were done with nude mice bearing a PC3 prostate xenograft. Liposomes containing Cy 5,5 were (after purification) administered into the mouse by tail vein injection in 300 µl volume. Mice were scanned by using Maestro fluorescence camera and the images were observed by using Maestro software. Biodistribution studies of the mouse were done one hour after injecting the Liposomal Cy 5,5. Organs were lysed and suspended in 1ml of PBS and transported to the 24 well plate. Fluorescence was measured using a Tecan Sapphire plate reader.

Liposomes have also been injected into nude mice having implanted prostate, breast, and melanoma tumors in addition to immunocompetent mice (with and without the ASM gene). These mice were imaged with liposomes loaded with PET agents (FDS loaded), MR agents (Gd), and optical agents (Luciferin).

### MRI studies

Mouse experiments were done with nude mice bearing PC3 prostate xenograft. Liposomes were separately loaded with multiple FDA approved contrast agents (for example, gadopentetate dimeglumine or gadolinium(III) 5,8-*bis*(carboxylatomethyl)-2-[2-(methylamino)-2-oxoethyl]-10-oxo-2,5,8,11-tetraazadodecane-1-carboxylate hydrate) were (after purification) administered into the mouse by tail vein injection in 300 µl volume. In some embodiments of the invention, the MR contrast agents were lyophilized and resuspended in Deuterium, which lacks the proton spin signal of endogenous water. The relaxative properties of the nanoparticles with agents suspended in H2O or D2O were compared using a table top relaxometer (Bruker mq20). The activation contrast of the nanoparticles after particle destabilization with Acid SMase was also determined.

For in vivo experiments, animals were subcutaneously implanted with murine fibrosarcomas were imaged with rodent MRI following administration of gadolinium and deuterium loaded sphingomyelin liposomes. The results were analyzed and viewed using conventional MRI software. After the initial imaging experiment, mice were subjected to varying doses of external beam X-ray irradiation and the activation of the liposomes by Acid SMase was read by further MR imaging. Control mice, which do not express the Acid SMase protein, and control nanoparticles (which do not contain sphingolipid) were found to produce no response.

### Results and Discussion

Liposome micelles and other nanoparticles can be used as a carrier of imaging tracers and therapeutic agents. Imaging results can then be used for diagnostic evidence-based patient management or for the development and optimization of drug delivery systems for therapy. The application of such platforms as vehicles for targeted delivery can be expanded by coupling of antibodies, peptides, and specific small molecules to the liposome surface. In this case, the present invention provides functionality to the liposome formulation by introducing sphingomyelin to the liposome formulation and then using it to release liposome content only in the presence of cells expressing sphingomyelinase outside of the cells.

The mechanism of action in the liposome of the present invention is that the sphingomyelin in the liposomes will be cleaved to the ceramide when in contact with the sphingomyelinase. The reaction product of acid SMase, ceramide, has been shown to form ceramide-enriched microdomains in both fluid and gel state phosphatidylcholine (PC) membranes. The segregated domains have physical properties very different from the membrane composed of phosphatidylcholine. Segregation of ceramide has been suggested to result from efficient intermolecular hydrogen bonding between the head groups of this lipid. The head group of ceramide is also weakly hydrated, which allows tight lateral packing in the membrane. Results conform with earlier observations, where SMase was injected onto the vesicle surface, resulting in domain formation and vectorial budding. Asymmetric formation of ceramide in a bilayer, i.e., either in the outer or inner leaflets, will cause an area difference between the two monolayers. Ceramide has also a tendency to form inverted nonlamellar phases.

These properties, area difference between adjacent monolayers, and negative spontaneous curvature for ceramide containing domains together with the high bending rigidity of the ceramide-enriched domain provide the driving force for vectorial formation of vesicles in the membrane.

### EXAMPLE II

### Liposome formulation

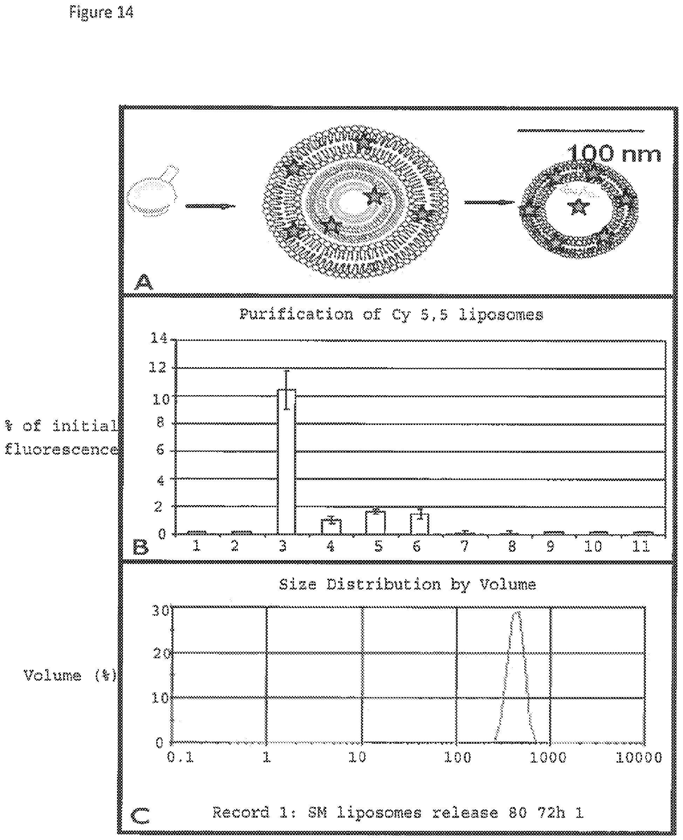

Lipids were purchased from Avanti Polar Lipids and Acid SMase produced by B. *cereus* from Sigma Aldrich. Liposomes were made by a lipid mixture consisting of a total of 20 uM of lipids in 4/3/3 DSPC/Cholesterol/SM in mol/mol ratios. Liposomes were prepared as described in Medina OP et al. Shortly, lipids stored in chloroform were pipetted to a round bottomed flask, dried under nitrogen and lyophilized for 2 h to remove trace amounts of chloroform. Lipids were allowed to hydrate for 30 min in 60 C buffered water solutions containing either Cy 5.5 or hairpin DNA or Alexa 680 streptavidin or radiolabeled albumin. Extrusion was performed 11 times through 100 nm polycarbon membrane by using a small volume extruder. Liposomes were purified from the unbound compounds by a PD 10 column. This revealed that Alexa 680, Cy 5 and BSA were relatively easy to encapsulate into the liposomes (Figs. 14A and B) Liposomes were controlled by liposome size measurements by using dynamic light scattering (Malvern) and revealed an average hydrodynamic diameter of 100-200 nm liposomes [95 % range?] (Fig 14C).

### In vitro assays

### Liposome activation assays

Liposomes containing Alexa 680 or Cy 5.5 were incubated after purification with different amounts of SMase as well as with heat inactivated SMase. Results were collected by using Tecan Sapphire plate reader at different time points.

Cy 5 is quenched when packed in liposomes. In theory, SMase treatment increases the signal by affecting to the bilayer of the liposomes and allowing the dye to be released. This was indeed observed upon incubation of Cy 5.5 loaded SM-containing liposomes with purified SMase. Thus SM liposomes were leaking after SMase was introduced to the liposomes detected by the increased fluorescence that could not be shown when heat treated SMase was implied (Fig 15A).

To obtain a valid correlation between SMase activity and signal intensity, a different system was used that exhibits significantly lower background signals, i.e. a DNA beacon containing a hairpin loop with a CY7-Dabcyl quenching pair was used instead of Cy 5.5 as payload. After the beacon was released from the liposomes by SMase to the medium outside which contained the corresponding target DNA interaction of beacon and target led to unquenching of the Cy 7 fluorophores. The resulting signals were measured with a Tecan Sapphire plate reader.

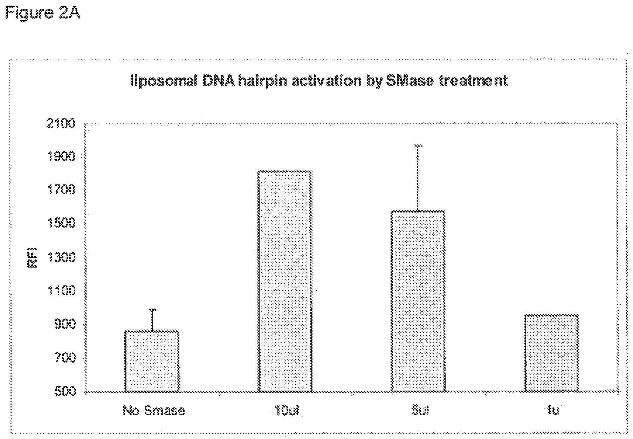

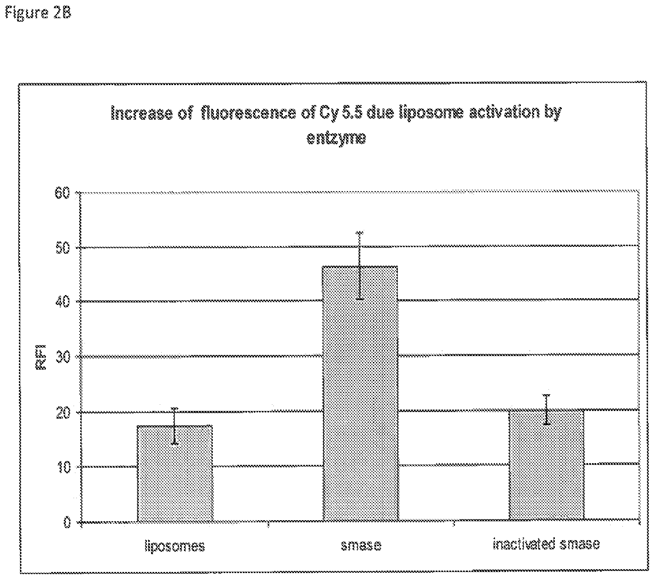

The general leakage as well as the linearity of the SMase-induced leakage was tested with liposomal CY7-Dabcyl hairpin encapsulated in SM liposomes while the target of the hairpin was located outside of the liposomes. When the target DNA hits the hairpin, the hairpin is lateralized and opened. This breaks the quenching giving free Cy7 fluorescence (Fig. 2B). We measured the opening of the CY7-Dabcyl hairpin released from the liposomes with and without addition of purified SMase in three different concentrations (1 U unite, 0.5 U unites and 0.1 U unites per ml), to outside medium containing target DNA. This experiment showed that there is linear correlation of SMase concentration and the cargo release in SM liposomes.

### SMase enzyme activity detection assay

SMase enzyme activity was visualized by a SMase detection kit (Cayman Chemicals, USA) according to the instructions provided. 10 µl of sample was added to at least three wells. To obtain reproducible results, the amount of sphingomyelinase added to the wells should fall within the range of the assay standard curve. When necessary, samples should be diluted with the SMase Acid Solution. Pipette samples together with positive and negative controls to a 96 well plate.

To initiate the reaction, add 20 µl of the diluted Sphingomyelin substrate prepared with SMase Acid Solution (Cayman Chemicals). Cover the plate with the plate cover and incubate at 37°C for thirty minutes. Add 100 µl of the 'Developer Solution' to all wells being used, including sample, sample background, positive control, and standards. Cover, and incubate for 30 minutes at 37°C. Remove the plate cover and read using an excitation wavelength of 530-540 nm and an emission wavelength of 585-595 nm. Primary human aortic endothelial cells (HAoEC) and PC-3 cells were irradiated with different doses ranging from 0 to 20 Gy. 10 min after irradiation, fluorescent signal intensity correlating with SMase enzyme activity were measured with a Tecan Sapphire plate reader.

To test the suitability of the cell model to the following experiments the SMase secretion was tested after inducing stress by radiation that may lead to apoptosis. The secretion of SMase after irradiation with various doses was detected by using SMase kit (Fig 2B). The PC-3 cells and the HAoEC secreted SMase in dose dependent manner from 5 Grays to 30 Grays showing very robust dose dependency to the radiation (Figs. 15C, D).

### In vivo experiments

### Tumor model

Athymic nude mice bearing PC3 prostate xenografts were used for in vivo evaluation of SM liposomes. 1,000,000 cells in 50 µl volume together with 50 µl matrigel each were injected subcutaneously into both the hind limbs. The animals were checked for tumor growth, weight loss and overall motility. Experiments were performed 3 weeks after injection.

In order to randomize the mice based on the tumor volume to exclude any size effects, tumor volumetry was performed with a Vevo 770 high frequency ultrasound device as previously described (*Hwang et al., 2010; Zhao et al., 2010*). The machine was equipped with 25 MHz and 55 MHz transducers, and a 3D motor on a rail system for automated acquisition of 3D stacks. The transducer position, gain settings, and midfield focus were initially optimized and maintained throughout each experiment. The mice were anesthetized by inhalation of 1-2% isoflurane (Baxter Healthcare, Deerfield, IL)/oxygen gas mixture and placed on a heated platform. The transducer was fixed with the rail system, and placed centrally above the tumor. Three-dimensional images in the B mode were acquired by computer controlled linear translation of the transducer over the whole length of the tumor by acquiring 2- dimensional images every 100 µm for morphologic analysis and tumor volumetry. 3 D image reconstruction was done automated by the Vevo 770 software (Visualsonics). All tumors showed some hypoechogenic areas consistent with necrosis. The tumor volumes varied between 500 mm³ and 3500 mm³ (Fig. 16A).

### Biodistribution studies

Radiolabeling of the albumin was performed using the IODOGEN method (Pierce) (10, 50). Specific activities of the 1241-bound particle fractions eluted from PD-10 columns were ~100-1,000 mCi/µm, assessed using a dose calibrator (Capintec) and radio-TLC. TLC plates were developed using a mixture of acetic acid/methanol (80:20 v/v) and dried, and the absorbance was analyzed by a TLC plate reader (Bioscan). Radiolabeled albumin was encapsulated in the liposomes in the hydration step. After 30 minutes hydration and extrusion 11 times through polycarbonate filter (100 nm pore size) the liposomes were purified by elution through PD-10 gel filtration column. Radiolabeled liposomes were administered via tail vein injection.

The biodistribution of Irradiated SM liposome treated mice vs. Irradiated PC liposome treated mice was done in 2h time point. Radio labeled BSA was used as payload inside of the liposomes (Fig. 16B).

### Tumor irradiation

The irradiation of the animals was done by X-ray irradiation of the sedated mice where only the tumors and the hind legs were exposed to the irradiation while the rest of the body was shielded by lead. The administered dose was 20 Gy. Optical imaging followed irradiation of the mice immediately.

### Fluorescence imaging

After purification, liposomes containing Alexa Fluor 680 fluorophores were administered by tail vein injection in a volume of 300 µl. The mice were imaged with a Maestro fluorescence camera. Image analysis was performed with the according Maestro software. Biodistribution measurements were done one hour after injections of the liposomal Alexa Fluor 680 fluorophore. Organs were lysed, suspended in 1ml of PBS, and transported to the 24 well plate. Fluorescence was measured by using Tecan Sapphire plate reader.

To further investigate the possibility to use the SM containing liposomes for detection of cell damage in vivo we used nude mice bearing subcutaneous PC-3 tumors. Each animal had two tumors one in each side of the hind leg.

One of the tumors was irradiated with 20 Grays while the other one was protected from irradiation by lead shielding. The ability of SM liposomes to deliver imaging markers was studied using optical imaging of irradiated nude mice bearing PC-3 tumors. Mice were injected with Alexa 680 containing liposomes prior to irradiation with 20 Grays followed by imaging with the Maestro. There was a significant homing of the Alexa Fluor 680 fluorophore to the tumors. The irradiated tumors showed considerable brighter signals than the non-irradiated tumors (16C). Images were taken immediately after irradiation, i.e. 30 minutes after injection of the liposomes (16D).

To investigate how much of this phenomenon is due the enhanced permeability and retention (EPR) effect and the leakage of the vasculature after irradiation we compared the SM liposomes to PC liposomes which should not have any active release or targeting function. The irradiation-imaging sequence was performed as described. The fluorescent signals in irradiated tumors were higher in the group that received SM liposomes than in the one that received PC liposomes indicating that the detected signals were generated to a significant amount by activation of the SM liposomes rather than non-specific accumulation due to enhanced vascular permeability after irradiation (Fig. 16C).

In order to determine whether the SMase impact was time dependent, the animals were imaged repeatedly 1 h, 4 h and 24 h after injection of the liposomes and irradiation of one tumor site according to the described protocol. The data showed the highest signals at early time points, as well as a steady clearance in all off the mice after several hours (Fig. 16).

The spectral analysis and quantification of Alexa 680 in tumors revealed that there were large significant differences in the amounts of Alexa 680 in the tumors depending of the radiation of the tumor and a liposome type that was used as a carrier. Sphingomyelin containing liposomes delivered Alexa 680 significantly better than sphingomyelin containing liposomes to the non-irradiated tumors or only PC containing liposomes to radiated or non-radiated tumors (Fig 16).

### Determination of main SMase source by fluorescence imaging

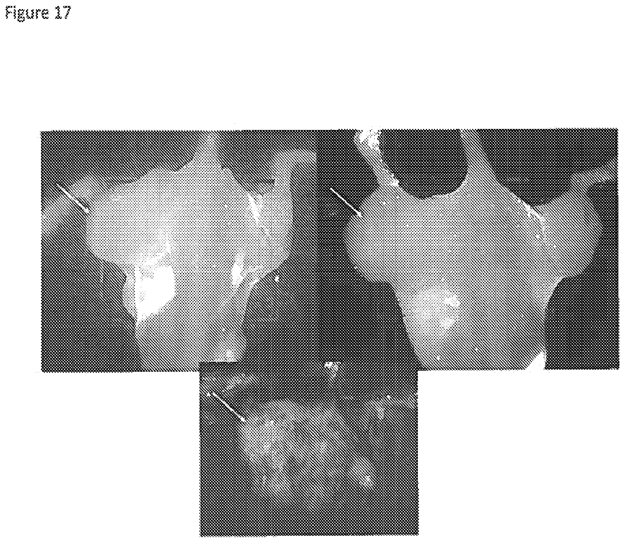

To further investigate the main cellular source of SMase, we used SMase knockout scid mice lacking SMase expression. Each animal had two tumors one in each side of the hind leg. The SMase knockout mice (Fig. 17) underwent the same experimental protocol. Only weak signals could be detected in either of the tumors.

The proposed mechanism of action in our liposomes is that the SM in the lipid layer will be cleaved to ceramide upon contact with SMase. Ceramide, the reaction product of SMase has been shown to form ceramide-enriched microdomains in both fluid and gel state phosphatidylcholine (PC) membranes. The segregated domains have physical properties very different from the membrane composed of phosphatidylcholine. It has been suggested that the segregation of ceramide results from efficient intermolecular hydrogen bonding between the head groups of this lipid. The head group of ceramide is also weakly hydrated, which allows tight lateral packing in the membrane. Our results build upon our earlier observations where SMase was injected onto the vesicle surface, resulting in domain formation and vectorial budding. Asymmetric formation of ceramide in a bilayer (i.e., either in the outer or inner leaflets) will cause an area difference between the two monolayers. Ceramide has also a tendency to form inverted nonlamellar phases. The area difference between adjacent monolayers and the negative spontaneous curvature for ceramide-containing domains are properties that should sufficiently result in increased leakiness of the liposome in the presence of ceramides.

There are several conditions where SMase has role like a variety of cell types present in atherosclerotic lesions secrete SMase, thus suggesting a possible role for this enzyme in the development of atherosclerosis so this imaging and targeting device could be possibly used also for diagnostics imaging and treatment of these diseases.

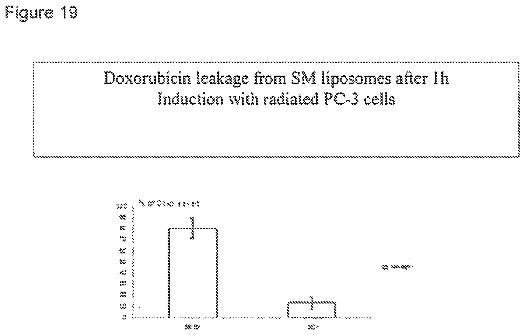

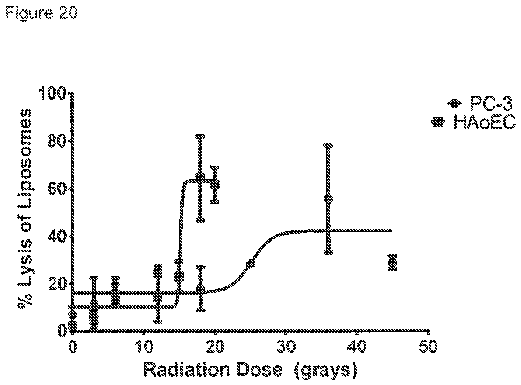

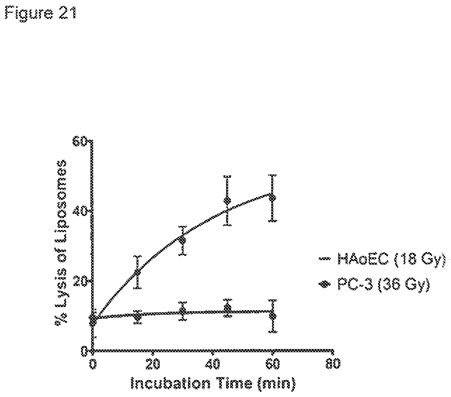

Here we showed that it is possible to design SM-containing liposomes that release their payload upon contact with SMase in vitro. Our study showed that PC-3 cells secrete SMase in dose dependent manner when irradiated from 5 Gr to 30 Gr. We also observed HAoEK endothelial cells secrete SMase in a similar manner when irradiated. Therefore, both endothelial cells and the cancer cells could be the source of the SMase during irradiation induced stress (see Figs. 19-21). Fluorescence in the SMase treated liposomes compared to non-SMase treated liposomes increases roughly in two-fold. This is due the release of Cy5.5 which is auto-quenched in liposomes when in close contact in high concentrations of each other. Moreover the inactivated SMase did not raise the fluorescence.

In vivo experiments all of the time points used in this study free Alexa 680 was rapidly cleared from the circulation and could be detected only in the bladder. But in the case of liposomal formulation there was clear homing of the liposomal Alexa 680 to the tumors. This was possibly due the passive EPR effects. However, there was a clear tendency of increased targeting of the fluorophore to the irradiated tumors in the case of SM containing liposomes. This could not be seen with PC liposomes or the SMase knockout mice even when tumors were irradiated. This suggests that there are SM specific mechanisms of the delivery of the payload to the tumors. When irradiated, the two possible sources for SMase secretion are the endothelial vessels of the tumors and the tumor cells. However, based on the results from the knockout mice, it seems more likely that the endothelial cells are the primary source for the SMase.

Liposomes, micelles and other nanoparticles can be used as a carrier of imaging tracers and imaging results can then be used for development and optimization of drug delivery systems for therapy. The applications of such platforms as vehicles for targeted delivery can be expanded by coupling of antibodies, peptides, and specific small molecules to the liposome surface. In this case we have introduced functionality to the liposome formulation by introducing SM to the liposome formulation and then using it to release liposome content only in the presence of cells expressing SMases outside of the cells. This can lead to a more beneficial pharmacodynamics profile of the drugs, DNA/RNA or potent peptides when compared to free drugs or liposomal delivery system alone. The SMase triggered liposome disruption could allow the drug to be administered before a radiation and the drug would be released only on the site of the radiation immediately when the radiation occurs. Because SMase secretion is not only limited in radiation this allow self-regulated delivery systems in other conditions, like apoptotic tissues or inflammation.

Some liposomal products are already FDA-approved for IV formulations, which should facilitate the translation of nano-delivery systems to clinical trials. These liposomal apoptosis triggered liposomes can be used for MRI imaging optical imaging, targeted drug delivery and gene delivery. In a theranostic approach, the imaging component can be used to first evaluate the drug delivery capabilities followed by treatment approaches.

## Claims

1. A liposomal system, comprising:
(a) a first layer comprising sphingomyelin or a derivative thereof having modified or shortened side chains;
(b) a second layer comprising sphingomyelin or a derivative thereof having modified or shortened side chains;
wherein the first and second layers form a bilayer liposome defining a hydrophilic interior compartment and a hydrophobic bilayer compartment;
(c) at least one therapeutic agent in the hydrophilic interior compartment or the hydrophobic bilayer compartment; and
(d) at least one imaging agent in the hydrophilic interior compartment or the hydrophobic bilayer compartment, wherein the imaging agent is: (i) quenched Cy 5,5, (ii) a DNA segment comprising a DNA Beacon containing a hairpin loop with a CY7-Dabcyl quenching pair or other fluorophor quencher pair, or (iii) gadolinium and deuterium (D₂O).

2. A liposomal system according to claim 1, wherein the therapeutic agent is a chemotherapeutic agent.

3. A liposomal system according to claim 1, wherein the imaging agent and the therapeutic agent are released from the liposomal system following contact of the liposomal system with SMase.

4. A liposomal system according to claim 1, in a pharmaceutically acceptable vehicle.

5. The liposomal system according to claim 1, wherein the imaging agent is unquenched following contact of the liposomal system with SMase.

6. The liposomal system according to claim 1, wherein the imaging agent is unquenched when it is released from the liposomal system.

7. The liposomal system according to claim 1, wherein the first and second layers comprise a mixture of the lipids DSPC/Cholesterol/Sphingomyelin in a 4/3/3 mol/mol ratio.

8. A method for delivering a therapeutic agent and an imaging agent, comprising:
contacting a liposomal system according to claim 1 with a cell sample under *in vitro* conditions wherein SMase present in or released by the cell sample can hydrolyze the sphingomyelin in the liposomal system to release therapeutic agent and the imaging agent from the liposomal system.

9. The method of claim 8, further comprising measuring a signal generated by the imaging agent.

10. The method of claim 9, wherein the signal generated by the imaging agent is used to monitor SMase activity in the cell sample.

11. The method of claim 9, wherein the signal generated by the imaging agent is used to determine cellular response to stress in the cell sample.

12. The method of claim 8, further comprising measuring a signal generated by the imaging agent as a readout for release of the therapeutic agent.

13. The method of claim 8, wherein the therapeutic agent is a chemotherapeutic agent.

## Patentansprüche

1. Liposomales System, umfassend:
(a) eine erste Schicht, umfassend Sphingomyelin oder ein Derivat davon mit modifizierten oder verkürzten Seitenketten;
(b) eine zweite Schicht, umfassend Sphingomyelin oder ein Derivat davon mit modifizierten oder verkürzten Seitenketten;
wobei die erste und die zweite Schicht ein Doppelschichtliposom bilden, das ein hydrophiles, inneres Kompartiment und ein hydrophobes Doppelschicht-Kompartiment definiert;
(c) mindestens ein therapeutisches Mittel in dem hydrophilen, inneren Kompartiment oder dem hydrophoben Doppelschicht-Kompartiment; und
(d) mindestens ein Bildgebungsmittel in dem hydrophilen, inneren Kompartiment oder dem hydrophoben Doppelschicht-Kompartiment, wobei das Bildgebungsmittel: (i) gequenchtes Cy 5,5, (ii) ein DNA-Segment, das einen DNA-Beacon umfasst, der eine Haamadelschleife mit einem CY7-Dabcyl-Quenchingpaar oder ein anderes Fluorophor-Quencherpaar enthält, oder (iii) Gadolinium und Deuterium (D₂O) ist.

2. Liposomales System nach Anspruch 1, wobei das therapeutische Mittel ein chemotherapeutisches Mittel ist.

3. Liposomales System nach Anspruch 1, wobei das Bildgebungsmittel und das therapeutische Mittel von dem liposomalen System nach Kontakt des liposomalen Systems mit SMase freigesetzt werden.

4. Liposomales System nach Anspruch 1, in einem pharmazeutisch verträglichen Träger.

5. Liposomales System nach Anspruch 1, wobei das Bildgebungsmittel nach Kontakt des liposomalen Systems mit SMase ungequencht wird.

6. Liposomales System nach Anspruch 1, wobei das Bildgebungsmittel ungequencht wird, wenn es von dem liposomalen System freigesetzt wird.

7. Liposomales System nach Anspruch 1, wobei die erste und die zweite Schicht eine Mischung der Lipide DSPC/Cholesterin/Sphingomyelin in einem molaren Verhältnis von 4/3/3 umfasst.

8. Verfahren zur Abgabe eines therapeutischen Mittels und eines Bildgebungsmittels, umfassend:
Inkontaktbringen eines liposomalen Systems nach Anspruch 1 mit einer Zellprobe unter In-vitro-Bedingungen, wobei in der Zellprobe vorliegende oder daraus freigesetzte SMase das Sphingomyelin in dem liposomalen System hydrolysieren kann, um das therapeutische Mittel und das Bildgebungsmittel aus dem liposomalen System freizusetzen.

9. Verfahren nach Anspruch 8, weiterhin umfassend das Messen eines von dem Bildgebungsmittel erzeugten Signals.

10. Verfahren nach Anspruch 9, wobei das von dem Bildgebungsmittel erzeugte Signal zum Überwachen der SMase-Aktivität in der Zellprobe verwendet wird.

11. Verfahren nach Anspruch 9, wobei das von dem Bildgebungsmittel erzeugte Signal zum Bestimmen der zellulären Reaktion auf Stress in der Zellprobe verwendet wird.

12. Verfahren nach Anspruch 8, weiterhin umfassend das Messen eines von dem Bildgebungsmittel erzeugten Signals als Anzeige für die Freisetzung des therapeutischen Mittels.

13. Verfahren nach Anspruch 8, wobei das therapeutische Mittel ein chemotherapeutisches Mittel ist.

## Revendications

1. Système liposomique, comprenant :
(a) une première couche comprenant de la sphingomyéline ou un de ses dérivés ayant des chaînes latérales modifiées ou raccourcies ;
(b) une seconde couche comprenant de la sphingomyéline ou un de ses dérivés ayant des chaînes latérales modifiées ou raccourcies ;
les première et seconde couches formant un liposome bicouche définissant un compartiment intérieur hydrophile et un compartiment de bicouche hydrophobe ;
(c) au moins un agent thérapeutique dans le compartiment intérieur hydrophile ou le compartiment de bicouche hydrophobe ; et
(d) au moins un agent d'imagerie dans le compartiment intérieur hydrophile ou le compartiment de bicouche hydrophobe, l'agent d'imagerie étant : (i) le Cy 5,5 désactivé, (ii) un segment d'ADN comprenant une balise d'ADN contenant une boucle en épingle à cheveux avec une paire de désactivation CY7-Dabcyl ou une autre paire de désactivateur fluorophore, ou (iii) du gadolinium et du deutérium (D₂O).

2. Système liposomique selon la revendication 1, dans lequel l'agent thérapeutique est un agent chimiothérapeutique.

3. Système liposomique selon la revendication 1, dans lequel l'agent d'imagerie et l'agent thérapeutique sont libérés du système liposomique après le contact du système liposomique avec la SMase.

4. Système liposomique selon la revendication 1, dans un véhicule pharmaceutiquement acceptable.

5. Système liposomique selon la revendication 1, dans lequel l'agent d'imagerie est activé après le contact du système liposomique avec la SMase.

6. Système liposomique selon la revendication 1, dans lequel l'agent d'imagerie est activé quand il est libéré du système liposomique.

7. Système liposomique selon la revendication 1, dans lequel les première et seconde couches comprennent un mélange des lipides DSPC/cholestérol/sphingomyéline dans un rapport moles/moles de 4/3/3.

8. Procédé de délivrance d'un agent thérapeutique et d'un agent d'imagerie, comprenant :
la mise en contact d'un système liposomique selon la revendication 1 avec un échantillon cellulaire dans des conditions in vitro la SMase présente dans ou libérée par l'échantillon cellulaire pouvant hydrolyser la sphingomyéline dans le système liposomique pour libérer l'agent thérapeutique et l'agent d'imagerie du système liposomique.

9. Procédé selon la revendication 8, comprenant en outre la mesure d'un signal produit par l'agent d'imagerie.

10. Procédé selon la revendication 9, dans lequel le signal produit par l'agent d'imagerie est utilisé pour suivre l'activité SMase dans l'échantillon cellulaire.

11. Procédé selon la revendication 9, dans lequel le signal produit par l'agent d'imagerie est utilisé pour déterminer la réponse cellulaire à un stress dans l'échantillon cellulaire.

12. Procédé selon la revendication 8, comprenant en outre la mesure d'un signal produit par l'agent d'imagerie comme lecture pour la libération de l'agent thérapeutique.

13. Procédé selon la revendication 8, dans lequel l'agent thérapeutique est un agent chimiothérapeutique.
